# EUROPEAN PATENT APPLICATION

(11) **EP 4 789 612 A1**
(43) Date of publication of application: **12.08.2026**
(21) Application number: 24834581.1
(22) Date of filing: 22.08.2024
(51) Int. Cl.: A61B 5/097, A61M 16/08, A61M 39/10

(54) **GLUE-FREE AND CHEMICAL-SOLVENT-FREE GAS SAMPLING TUBE AND RESPIRATORY THERAPY DEVICE**

(30) Priority: 16.08.2024 CN 202411130826
(71) Applicant: Vincent Medical (Dong Guan) Technology Co., Ltd, Dongguan, Guangdong 523808 (CN); Vincent Medical Admired Co., Ltd., Dongguan, Guangdong 523000 (CN); Vincent Medical (Dong Guan) Manufacturing Co., Ltd., Dongguan, Guangdong 523730 (CN)
(72) Inventor: NG, Kam Man, Dongguan, Guangdong 523730 (CN); CHOI, Cheung Tai Raymond, Dongguan, Guangdong 523730 (CN); LI, Siu Tung, Dongguan, Guangdong 523808 (CN); FENG, Zhiwei, Dongguan, Guangdong 523808 (CN)
(74) Representative: KIPA AB
(86) International application number: PCT/CN2024/114046
(87) International publication number: WO 2026/036429

(57) **Abstract**

A glue-free chemical solvent-free gas sampling tube and respiratory therapy equipment are provided, which relate to the technical field of respiratory therapy equipment. The gas sampling tube includes: a gas delivery tube; a gas sampling tube connector, where the gas delivery tube is arranged on the gas sampling tube connector; and a locking fastener, where the locking fastener is configured to fix the gas delivery tube to the gas sampling tube connector. The gas sampling tube replaces in a solution of connecting the gas sampling tube connector and the gas delivery tube through glue or a chemical solvent in a conventional art by achieving a stable connection between the gas sampling tube connector and the gas delivery tube through the additionally arranged locking fastener, and then a novel glue-free chemical solvent-free gas sampling tube is provided, so that release of volatile organic matters is avoided fundamentally, meanwhile, the gas sampling tube connector is mechanically connected to the gas delivery tube by using the locking fastener without waiting for glue to air-dry and cure, manufacturing time of the gas sampling tube is shortened, and production capacity of the gas sampling tube is improved.

## Description

### TECHNICAL FIELD

The present disclosure relates to the technical field of respiratory therapy equipment, and in particular, to a glue-free chemical solvent-free gas sampling tube and respiratory therapy equipment.

### BACKGROUND

At present, an assembly process fora gas sampling tube generally adopts glue for curing and connecting. To ensure stable curing and connecting of the glue, the assembly process for the gas sampling tube usually needs to be air-dried for a long time or irradiated by using UV light to accelerate curing.

However, after assembly is completed, an existing glue curing process has a problem of volatilization of volatile organic matters, which poses a safety risk for a patient when using the gas sampling tube.

Therefore, a conventional art has defects and disadvantages, which needs to be further improved and developed.

### SUMMARY

In view of the foregoing disadvantages in a conventional art, an objective of the present disclosure is to provide a glue-free chemical solvent-free gas sampling tube and respiratory therapy equipment, which aims to solve a problem of volatilization of volatile organic matters in an existing assembly process for a gas sampling tube in the conventional art.

A technical solution adopted in the present disclosure for solving a technical problem is as follows: a glue-free chemical solvent-free gas sampling tube, configured for a respiratory therapy equipment. The gas sampling tube includes:
a gas delivery tube;
a gas sampling tube connector, where the gas delivery tube is arranged on the gas sampling tube connector; and
a locking fastener, where the locking fastener is configured to fix the gas delivery tube to the gas sampling tube connector.

Optionally, the gas sampling tube connector has a first inverted fastener, a first tube entrance piece, a locking part, and a first sleeving piece arranged in sequence from top to bottom; and the first sleeving piece is sleeved over the respiratory therapy equipment, and the first tube entrance piece is detachably connected to the gas delivery tube.

The locking fastener is arranged as a connector locking fastener, the connector locking fastener is provided with a perforation hole, a plurality of fixing pieces are fixedly arranged on the connector locking fastener, and the plurality of fixing pieces are configured to cooperate with the locking part and the gas sampling tube connector to fix the gas delivery tube to an interior of the first tube entrance piece.

Optionally, each of the plurality of locking parts includes:
a propping boss, where the propping boss is provided below the first tube entrance piece, and the propping boss is provided above the first sleeving piece; and
an anti-stuck bulge, where the anti-stuck bulge is provided at an end of the propping boss deviating from the first tube entrance piece, and the anti-stuck bulge is integrally formed with the first sleeving piece and the propping boss; and the anti-stuck bulge is configured to partition a space between the propping bulge and the first sleeving piece into a clamping area.

Optionally, an end of the propping boss close to the first tube entrance piece is provided with an expansion part, and the expansion part is configured to expand the plurality of fixing pieces outwards; and an included angle between a tangent line of the expansion part and an axis of the gas sampling tube connector is set as 20°-80°.

Optionally, each of the plurality of fixing pieces includes a plurality of clinching fasteners, and the plurality of clinching fasteners are fixedly arranged at an end of the connector locking fastener deviating from the perforation hole; and each of the plurality of fixing pieces includes a deformation part and a buckle part.

Optionally, the buckle part is integrally provided with a propping section, a side support section, and a locking section, and an included angle between a tangent line of the propping section and a tangent line of the locking section is set as 10°-90°. The gas delivery tube penetrates out from the perforation hole, and the gas delivery tube is sleeved in the first inverted fastener and the first tube entrance piece, so that the connector locking fastener displaces to a direction of the gas sampling tube connector, the propping section is propped against the expansion part, the expansion part expands outwards to drive the deformation part to bend, and the side support section is propped against a side wall of the propping boss to lock the locking section in the clamping area.

Optionally, the connector locking fastener further includes:
a first ultrasonic line, where the first ultrasonic line is annularly arranged at an end of the connector locking fastener deviating from the perforation hole, and the first ultrasonic line is configured to perform ultrasonic welding with the gas sampling tube connector.

Optionally, a cross section of the first ultrasonic line along an axial direction of the connector locking fastener is arranged in a conical shape, a triangle, or a cone shape.

Optionally, a diameter of the first ultrasonic line is set as 1.1 times-2 times that of the perforation hole.

Optionally, each of the plurality of locking parts includes a locking boss, and the locking boss is fixedly provided above the first tube entrance piece.

Each of the plurality of fixing pieces includes a plurality of rotary fasteners, the plurality of rotary fasteners are fixedly arranged on an inner side wall of the connector locking fastener, and the plurality of rotary fasteners are configured to cooperate with the locking boss and the gas sampling tube connector to fix the gas delivery tube to the first tube entrance piece.

The gas delivery tube penetrates out from the perforation hole, the gas delivery tube is sleeved in the first inverted fastener and the first tube entrance piece, the connector locking fastener is propped against the locking boss, the plurality of rotary fasteners are rotated into the locking boss by rotating the connector locking fastener, and ultrasonic welding is performed on the locking boss and the gas sampling tube connector by using the first ultrasonic line.

Optionally, the gas sampling tube connector has a second inverted fastener, a second tube entrance piece, and a second sleeving piece arranged in sequence from top to bottom; and the second sleeving piece is sleeved over the respiratory therapy equipment, and the second tube entrance piece is detachably connected to the gas delivery tube.

The locking fastener is arranged as a clamping fastener, where the clamping fastener is provided with a first clamping piece, a second clamping piece, and a deformation connecting part in sequence, each of the first clamping piece and the second clamping piece is provided with a semicircular groove, and a shape of the semicircular groove is adapted to the gas delivery tube; the deformation connecting part is rotatably connected to the first clamping piece and the second clamping piece; and the clamping fastener is configured to fix the gas delivery tube to interiors of the second inverted fastener and the second tube entrance piece.

Optionally, the deformation connecting part is provided with a first connecting part, a deformation main body, and a second connecting part. The first connecting part is fixedly connected to the first clamping piece. The second connecting part is fixedly connected to the second clamping piece. Each of the connecting part and the second connecting part is rotatably connected to the deformation main body.

Optionally, a bending groove is provided at a rotatable connection position between the first connecting part and the deformation main body and between the second connecting part and the deformation main body.

Optionally, a clamping bulge is provided on the first clamping piece, and a clamping buckle is arranged on the second clamping piece.

The clamping buckle is buckled in the clamping bulge by rotating the second clamping piece in a case that the semicircular groove of the first clamping piece fits the gas delivery tube.

Optionally, an outer side surface of the first clamping piece corresponding to the second clamping piece is provided with a second ultrasonic line, and the second ultrasonic line is configured to perform ultrasonic welding on the first clamping piece and the second clamping piece.

Another technical solution adopted in the present disclosure for solving the technical problem is as follows: respiratory therapy equipment includes the glue-free chemical solvent-free gas sampling tube as described above.

Compared with a conventional art, the present disclosure provides a glue-free chemical solvent-free gas sampling tube and respiratory therapy equipment. The gas sampling tube replaces in a solution of connecting the gas sampling tube connector and the gas delivery tube through glue or a chemical solvent in the conventional art by achieving a stable connection between the gas sampling tube connector and the gas delivery tube through the additionally arranged locking fastener, and then a novel glue-free chemical solvent-free gas sampling tube is provided, so that release of volatile organic matters is avoided fundamentally, a production process is simplified, and safety and reliability of a gas delivery tube are significantly improved, meanwhile, the gas sampling tube connector is mechanically connected to the gas delivery tube by using the locking fastener without waiting for glue to air-dry and cure, manufacturing time of the gas sampling tube is shortened, and production capacity of the gas sampling tube is improved.

### BRIEF DESCRIPTION OF THE DRAWINGS

FIG. 1 is a schematic diagram of a three-dimensional structure of a glue-free chemical solvent-free gas sampling tube according to the present disclosure.
FIG. 2 is an exploded view of a three-dimensional structure of a gas sampling tube connector and a connector locking fastener of a glue-free chemical solvent-free gas sampling tube according to the present disclosure.
FIG. 3 is a front view of a three-dimensional structure of a gas sampling tube connector of a glue-free chemical solvent-free gas sampling tube according to the present disclosure.
FIG. 4 is a front view of a connector locking fastener of a glue-free chemical solvent-free gas sampling tube according to the present disclosure.
FIG. 5 is a front view of a glue-free chemical solvent-free gas sampling tube according to the present disclosure.
FIG. 6 is a cross-sectional view of FIG. 5 in I-I direction.
FIG. 7 is another exploded view of a three-dimensional structure of a glue-free chemical solvent-free gas sampling tube according to the present disclosure.
FIG. 8 is another exploded view of a three-dimensional structure of a glue-free chemical solvent-free gas sampling tube according to the present disclosure.
FIG. 9 is an exploded view of a three-dimensional structure of a locking fastener and a gas sampling tube connector of a glue-free chemical solvent-free gas sampling tube according to the present disclosure.
FIG. 10 is a schematic diagram of a three-dimensional structure of a clamping locking fastener of a glue-free chemical solvent-free gas sampling tube according to the present disclosure.
FIG. 11 is a schematic diagram of a three-dimensional structure of the clamping locking fastener of a glue-free chemical solvent-free gas sampling tube according to the present disclosure from another perspective.
FIG. 12 is another exploded view of a three-dimensional structure of a glue-free chemical solvent-free gas sampling tube according to the present disclosure.
FIG. 13 is another schematic diagram of a three-dimensional structure of a glue-free chemical solvent-free gas sampling tube according to the present disclosure.

Reference signs in the drawings:
10, gas sampling tube; 11, gas delivery tube; 12, gas sampling tube connector; 121, first inverted fastener; 122, first tube entrance piece; 123, locking part; 1231, propping boss; 1232, anti-stuck boss; 1233, expansion part; 1234, locking boss; 124, first sleeving piece; 13, connector locking fastener; 131, perforation hole; 132, fixing piece; 1321, clinching fastener; 1322, deformation part; 1323, buckle part; 1324, propping section; 1325, side support section; 1326, locking section; 1327, rotary fastener; 125, second inverted fastener; 126, second tube entrance piece; 127, second sleeving piece; 14, locking fastener; 141, clamping locking fastener; 1411, first clamping piece; 1412, second clamping piece; 1413, deformation connecting part; 1414, semicircular groove; 1415, first connecting part; 1416, deformation main body; 1417, second connecting part; 1418, bending groove; 1419, clamping bulge; 1420, clamping buckle; and 1421, second ultrasonic line.

### DETAILED DESCRIPTION OF THE EMBODIMENTS

Embodiments of the present disclosure are described in detail below, and examples of the embodiments are shown in accompanying drawings, where the same or similar reference numerals throughout the description represent the same or similar elements or the elements having the same or similar functions. The embodiments described below with reference to the accompanying drawings are exemplary, and are only used for explaining the present disclosure and cannot be construed as a limitation to the present disclosure.

In descriptions of the present disclosure, it is to be understood that orientations or positional relationships indicated by terms "center", "longitudinal", "transverse", "upper", "lower", "front", "rear", "left", "right", "vertical", "horizontal", "top", "bottom", "inner", "outer", and the like are the orientations or positional relationships shown based on the accompanying drawings, and are merely for the convenience of describing the present disclosure and simplifying the descriptions, rather than indicating or implying that the devices or elements referred to need to have particular orientations, and constructed and operated in particular orientations. Therefore, it cannot be construed as a limitation to the present disclosure. In addition, terms "first" and "second" are used merely for the purpose of description, and cannot be construed as indicating or implying relative importance or implying a quantity of indicated technical features. Therefore, a feature limited by "first" or "second" may explicitly or implicitly include one or more of the features. In the descriptions of the present disclosure, unless otherwise specified, "a plurality of" means two or more.

In the descriptions of the present disclosure, it is to be noted that, unless otherwise specified and limited, terms "mount", "interconnect", and "connect" are to be broadly understood. For example, the terms may refer to fixed connection and may also refer to detachable connection or integration; the terms may refer to mechanical connection and may alternatively refer to electrical connection; the terms may refer to direct mutual connection, may alternatively refer to indirect connection through a medium, and may refer to communication in two components. For those of ordinary skill in the art, specific meanings of the foregoing terms in the present disclosure may be understood according to specific situations.

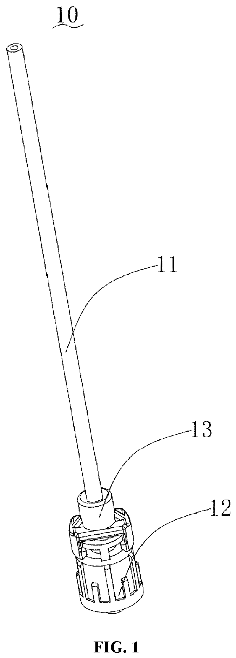

Referring to FIG. 1 to FIG. 3, a first embodiment of the present disclosure provides a glue-free chemical solvent-free gas sampling tube 10, configured for respiratory therapy equipment. The gas sampling tube includes a gas delivery tube 11, a gas sampling tube connector 12, and a locking fastener 14. The gas sampling tube 10 replaces in a solution of connecting the gas sampling tube 10 connector and the gas delivery tube 11 through glue or a chemical solvent in the conventional art by achieving a stable connection between the gas sampling tube 10 connector and the gas delivery tube 11 through the additionally arranged locking fastener 14, and then a novel glue-free chemical solvent-free gas sampling tube 10 is provided, so that release of volatile organic matters is avoided fundamentally, a production process is simplified, and safety and reliability of a gas delivery tube are significantly improved, meanwhile, the gas sampling tube 10 connector is mechanically connected to the gas delivery tube 11 by using the locking fastener 14 without waiting for glue to air-dry and cure, manufacturing time of the gas sampling tube 10 is shortened, and production capacity of the gas sampling tube 10 is improved.

Referring to FIG. 1 to FIG. 3, in some embodiments, the gas sampling tube 10 includes a gas delivery tube 11, a gas sampling tube connector 12, and a connector locking fastener 13. In an actual assembly process, one end of the gas delivery tube 11 penetrates through the perforation hole 131 of the connector locking fastener 13 first, and then the gas delivery tube 11 is inserted into the first inverted fastener 121 and the first tube entrance piece 122. The first inverted fastener 121 can prevent the gas delivery tube 11 from accidentally separating from the first tube entrance piece 122. A plurality of fixing pieces 132 arranged on the connector locking fastener 13 are configured to firmly fix the gas delivery tube 11 to an interior of the first tube entrance piece 122. In a case that the gas delivery tube 11 is inserted into the first tube entrance piece 122, the propping boss 1231 below the first tube entrance piece 122 cooperates with the anti-stuck boss 1232 to form a clamping area, so as to ensure stable positioning of the gas delivery tube 11 during assembling, and prevent the gas delivery tube 11 from falling off or loosening. In a case that the connector locking fastener 13 displaces downwards and is buckled in the clamping area, the fixing pieces 132 are expanded outwards through an expansion part 1233, so as to further ensure a stable connection of the gas delivery tube 11. A clinching fastener 1321 includes a deformation part 1322 and a buckle part 1323. The buckle part 1323 is provided with a propping section 1324, a side support section 1325, and a locking section 1326. In a case that the connector locking fastener 13 is buckled downwards, the deformation part 1322 of the clinching fastener 1321 is bent, and the propping section 1324 of the buckle part 1323 is first in contact with the expansion part 1233, so that the fixing pieces 132 are gradually expanded. With the connector locking fastener 13 continues to be pressed downwards, the side support section 1325 is propped against a side wall of the propping boss 1231 to provide an additional support force to further enhance connection stability. Finally, the locking section 1326 enters the clamping area and is locked to firmly fix the gas delivery tube 11 to interiors of the first inverted fastener 121 and the first tube entrance piece 122 of the gas sampling tube connector 12, and finally, the gas delivery tube 11 is firmly connected to the gas sampling tube connector 12 without loosening. In this mechanical connection manner, a chemical bonding agent does not need to be used in an overall assembly process. After assembly is completed, the gas sampling tube 10 has good air tightness, gas leakage is avoided, a production process is simplified, and safety and reliability of a product are significantly improved. According to the gas sampling tube 10, a traditional glue curing process is replaced by a mechanical connection manner, so that release of volatile organic matters is avoided fundamentally, safety of the gas sampling tube 10 is improved, meanwhile, a mechanical connection process is used without waiting for glue to air-dry and cure, manufacturing time of the gas sampling tube 10 is shortened, and production capacity of the gas sampling tube 10 is improved.

Referring to FIG. 1 to FIG. 3, in some embodiments, the gas sampling tube 10 includes a gas delivery tube 11, a gas sampling tube connector 12, and a connector locking fastener 13. The gas delivery tube 11 is configured to delivery or output gas. The gas sampling tube connector 12 is detachably connected to respiratory therapy equipment. The connector locking fastener 13 is configured to cooperate with the gas sampling tube connector 12 to fix the gas delivery tube 11 to an interior of the gas sampling tube 10. The gas sampling tube connector 12 has a first inverted fastener 121, a first tube entrance piece 122, a locking part 123, and a first sleeving piece 124. The first inverted fastener 121, the first tube entrance piece 122, the locking part 123, and the first sleeving piece 124 are arranged in sequence from top to bottom. The first sleeving piece 124 is sleeved over the respiratory therapy equipment. The first tube entrance piece 122 is detachably connected to the gas delivery tube 11. The connector locking fastener 13 is provided with a perforation hole 131. A hole diameter of the perforation hole 131 is slightly greater than a hole diameter of the gas delivery tube 11, so as to ensure that the gas delivery tube 11 penetrates out from the perforation hole 131 smoothly. A plurality of fixing pieces 132 are fixedly arranged on the connector locking fastener 13, and the plurality of fixing pieces 132 are configured to cooperate with the locking part 123 and the gas sampling tube connector 12 to fix the gas delivery tube 11 to an interior of the first tube entrance piece 122, so that the gas sampling tube 10 uses a mechanical connection manner to replace a traditional glue curing process, release of volatile organic matters is avoided fundamentally, safety of the gas sampling tube 10 is improved, meanwhile, a mechanical connection process is used without waiting for glue to air-dry and cure, manufacturing time of the gas sampling tube 10 is shortened, and production capacity of the gas sampling tube 10 is improved.

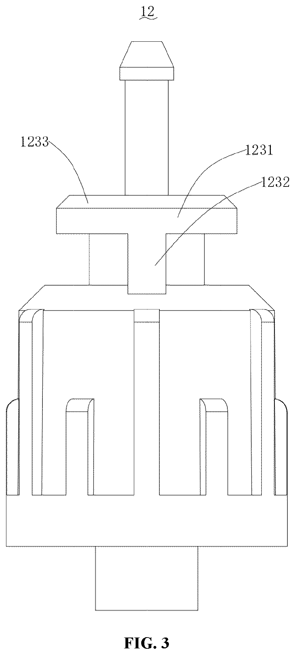

Referring to FIG. 3, in some embodiments, each of the plurality of locking parts 123 includes a propping boss 1231 and an anti-stuck bulge 1232. The propping boss 1231 is provided below the first tube entrance piece 122, and the propping boss 1231 is provided above the first sleeving piece 124, that is, the propping boss 1231 is arranged between the first tube entrance piece 122 and the first sleeving piece 124, which is beneficial to sleeving the gas delivery tube 11 in the first tube entrance piece 122. The anti-stuck bulge 1232 is provided at an end of the propping boss 1231 deviating from the first tube entrance piece 122, and the anti-stuck bulge 1232 is integrally formed with the first sleeving piece 124 and the propping boss 1231. The first sleeving piece 124 is connected to the propping boss 1231 through the anti-stuck bulge 1232, so that overall structural stability of the first sleeving piece 124 and the propping boss 1231 is enhanced, and the first sleeving piece 124 and the propping boss 1231 can bear a greater external force and impact. The anti-stuck bulge 1232 is configured to partition a space between the propping bulge 1231 and the first sleeving piece 124 into a clamping area, so that a clear clamping path and position are provided for the connector locking fastener 13, and risks of misaligned clamping and accidental falling off of the connector locking fastener 13 are avoided.

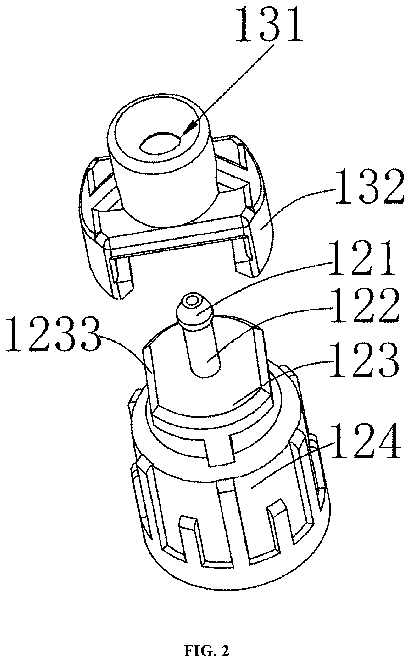

Referring to FIG. 2 and FIG. 3, in some embodiments, the propping bulge 1231 is provided with an expansion part 1233. The expansion part 1233 is provided at an end close to the first tube entrance piece 122. The expansion part 1233 is configured to expand the plurality of fixing pieces 132 outwards, which helps the fixing pieces 132 to expand outwards under an action of an external force in a process of clamping the fixing pieces 132. The plurality of fixing pieces 132 are adapted to the propping bulges 1231 in different sizes and shapes, so that compatibility and flexibility of a mechanical connection between the connector locking fastener 13 and the gas sampling tube connector 12 are improved. An included angle between a tangent line of the expansion part 1233 and an axis of the gas sampling tube connector 12 is set as 20°-80°. The included angle between the tangent line of the expansion part 1233 and the axis of the gas sampling tube connector 12 may be set as 20°, 30°, and 80°, and the fixing pieces 132 are uniformly stressed during expanding, and risks of damage or failure of the fixing pieces 132 caused by stress concentration of the fixing pieces 132 are avoided.

Referring to FIG. 2 and FIG. 3, in some embodiments, each of the plurality of fixing pieces 132 includes a plurality of clinching fasteners 1321. The plurality of clinching fasteners 1321 are fixedly arranged at an end of the connector locking fastener 13 deviating from the perforation hole 131. Each of the plurality of fixing pieces 1321 includes a deformation part 1322 and a buckle part 1323. The deformation part 1322 is configured to deform temporarily in a case that the buckle part 1323 is subjected to an external force, and returns to an original shape in a case that the buckle part 1323 is not subjected to an external force or the external force disappears, so that the connector locking fastener 13 can achieve better deformation and locking effects during clamping, and stability and reliability of a connection between the connector locking fastener 13 and the locking part 123 are ensured.

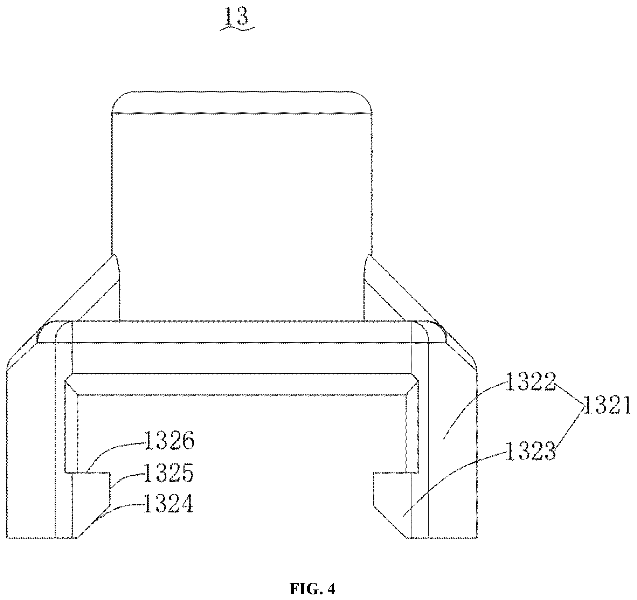

Referring to FIG. 4, in some embodiments, the buckle part 1323 is provided with a propping section 1324, a side support section 1325, and a locking section 1326. The propping section 1324, the side support section 1325, and the locking section 1326 are arranged integrally, so that the connector locking fastener 13 can be ensured to be accurately clamped into the clamping area through a cooperative effect of the propping section 1324, the side support section 1325, and the locking section 1326, and a stable locking effect can be achieved. An included angle between a tangent line of the propping section 1324 and a tangent line of the locking section 1326 is set as 10°-90°. The included angle between the tangent line of the propping section 1324 and the tangent line of the locking section 1326 is specifically set as 10°, 60°, or 90°, so that smooth transition of the buckle part 1323 from initial propping to final locking is achieved, and accuracy and stability of a connection between the connector locking fastener 13 and the locking part 123 are improved. The gas delivery tube 11 penetrates out from the perforation hole 131, and the gas delivery tube 11 is sleeved in the first inverted fastener 121 and the first tube entrance piece 122, so that the connector locking fastener 13 displaces to a direction of the gas sampling tube connector 12, the propping section 1324 is propped against the expansion part 1233, the expansion part 1233 expands outwards to drive the deformation part 1322 to bend, and the side support section 1325 is propped against a side wall of the propping boss 1231 to lock the locking section 1326 in the clamping area. In this mechanical connection manner, a chemical bonding agent does not need to be used in an overall assembly process. After assembly is completed, the gas sampling tube 10 has good air tightness, gas leakage is avoided, a production process is simplified, and safety and reliability of a product are improved. The gas sampling tube 10 adopts a mechanical connection manner to replace a traditional glue curing process, release of volatile organic matters is avoided fundamentally, safety of the gas sampling tube 10 is improved, meanwhile, a mechanical connection process is used without waiting for glue to air-dry and cure, manufacturing time of the gas sampling tube 10 is shortened, and production capacity of the gas sampling tube 10 is improved.

In some embodiments, the connector locking fastener 13 further includes a first ultrasonic line. The first ultrasonic line is annularly arranged at an end of the connector locking fastener 13 deviating from the perforation hole 131, and the first ultrasonic line is configured to perform ultrasonic welding with the gas sampling tube connector 12, so that a contact surface between the connector locking fastener 13 and the gas sampling tube connector 12 is molten and recombined under high temperature and pressure to form a seamless connection, air tightness of the connection is effectively improved, a risk of gas leakage is reduced, meanwhile, ultrasonic welding may be directly performed during assembling the gas sampling tube 10, additional glue curing time or UV light irradiation are not required, an assembly process is further simplified, and production efficiency of the gas sampling tube 10 is improved.

In some embodiments, a cross section of the first ultrasonic line along an axial direction of the connector locking fastener 13 is arranged in a conical shape, a triangle, or a cone shape, which is beneficial for propagation and focusing of ultrasonic waves during welding, and welding efficiency and quality are improved. These shapes can better guide ultrasonic energy, so that uniform welding points are formed on the contact surface.

In some embodiments, a diameter of the first ultrasonic line is set as 1.1 times-2 times that of the perforation hole 131, that is, the first ultrasonic line is arranged on an outer side of the water inlet hole, thereby optimizing a welding effect between the gas sampling tube connector 12 and the connector locking fastener 13, ensuring strength and air tightness of a connection position between the gas sampling tube connector 12 and the connector locking fastener 13, and improving reliability of an overall structure. The diameter of the first ultrasonic line is greater than that of the perforation hole 131, which can uniformly transfer stress during welding, a stress concentration phenomenon is reduced, and anti-fatigue performance of the connection position between the gas sampling tube connector 12 and the connector locking fastener 13 is improved.

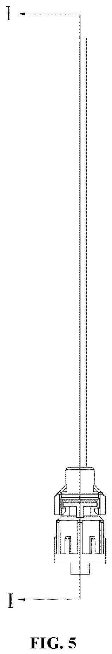

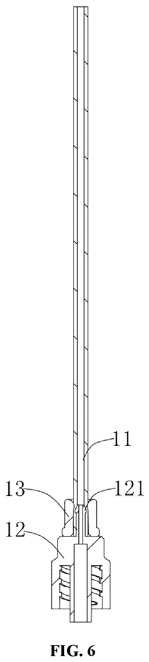

Referring to FIG. 5 and FIG. 6, in some embodiments, the first inverted fastener 121 is arranged in a cone shape. A maximum diameter of the first inverted fastener 121 is slightly greater than that of the gas delivery tube 11, so that the gas delivery tube 11 is effectively preventing from being unplugged from the first inverted fastener 121 after being stressed, and assembly efficiency between the gas delivery tube 11 and the gas sampling tube connector 12.

In some embodiments, the sharper the tip of the first ultrasonic line, the better, so that vibration energy may be concentrated at the tip of the triangle, then a uniform plastic melt flow is formed on an overall welding interface by accumulated heat, and welding efficiency and quality are improved, a connection between the gas sampling tube connector 12 and the connector locking fastener 13 is more stable, a curing process using glue is abandoned, a safety risk caused by volatilization of volatile organic matters is eliminated, user safety is ensured, air-drying curing time is not required, manufacturing time and cost for the gas sampling tube 10 are reduced, and production capacity of products of the gas sampling tube 10 is improved.

In some embodiments, a welding principle of the first ultrasonic line is that high-frequency friction is performed on a contact surface between the gas sampling tube connector 12 and the connector locking fastener 13 to convert mechanical energy into thermal energy by using high-frequency vibration of a welding head of the first ultrasonic line, the thermal energy dissolves molecules on a welding surface between the gas sampling tube connector 12 and the connector locking fastener 13 to recover activity of the molecules, then the molecules are entangled with each other to achieve a welding purpose under assistance of an external acting force, air tightness and welding quality between the gas sampling tube connector 12 and the connector locking fastener 13 are greatly improved, a problem of loosening that may be caused by a traditional glue connection is avoided, a curing process using glue is abandoned, a safety risk caused by volatilization of volatile organic matters is eliminated, user safety is ensured, equipment safety is improved, air-drying curing time is not required, manufacturing time and cost for the gas sampling tube 10 are reduced, and production capacity of products of the gas sampling tube 10 is improved.

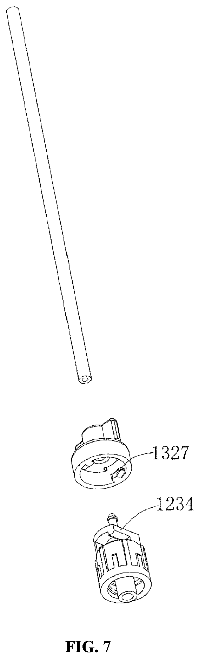

Referring to FIG. 7, in some embodiments, each of the plurality of locking parts 123 includes a locking boss 1234, and the locking boss 1234 is fixedly provided above the first tube entrance piece 122, so that the gas sampling tube connector 12 can be quickly mounted and fixed, an assembly process is simplified, and production efficiency is improved.

Referring to FIG. 7, in some embodiments, each of the plurality of fixing pieces 132 includes a plurality of rotary fasteners 1327. The plurality of rotary fasteners 1327 are fixedly arranged on an inner side wall of the connector locking fastener 13, and the plurality of rotary fasteners 1327 are configured to cooperate with the locking boss 1234 and the gas sampling tube connector 12 to fix the gas delivery tube 11 to the first tube entrance piece 122. The gas delivery tube 11 penetrates out from the perforation hole 131, the gas delivery tube 11 is sleeved in the first inverted fastener 121 and the first tube entrance piece 122, the connector locking fastener 13 is propped against the locking boss 1234, the plurality of rotary fasteners 1327 are rotated into the locking boss 1234 by rotating the connector locking fastener 13, and ultrasonic welding is performed on the locking boss 1234 and the gas sampling tube connector 12 by using the first ultrasonic line to provide double locking for fixation of the gas delivery tube 11, which ensures that the gas delivery tube 11 dose not loosen or leak during using. Meanwhile, reliability and efficiency during assembling are improved, and service life of equipment is prolonged. Meanwhile, in this mechanical connection manner, a chemical bonding agent does not need to be used in the overall assembling process. After assembly is completed, the gas sampling tube 10 has good air tightness, gas leakage is avoided, a production process is simplified, safety and reliability of the product are significantly improved, the gas sampling tube 10 adopts a mechanical connection manner to replace a traditional glue curing process, release of volatile organic matters is avoided fundamentally, safety of the gas sampling tube 10 is improved, meanwhile, a mechanical connection process is used without waiting for glue to air-dry and cure, manufacturing time of the gas sampling tube 10 is shortened, and production capacity of the gas sampling tube 10 is improved.

In some embodiments, an outer surface of the first sleeving piece 124 is provided with a plurality of anti-slip bulges. The plurality of anti-slip bulges are arranged at intervals, and a frictional force of the gas sampling tube connector 12 is increased, which helps to shorten assembly time between the gas sampling tube 10 and respiratory therapy equipment, further shortens manufacturing time of the respiratory therapy equipment, and improves production capacity of the respiratory therapy equipment.

In some embodiments, an inner surface of the first sleeving piece 124 is provided with screw threads. The screw threads are configured for threaded connection with the respiratory therapy equipment, which improves air tightness between the gas sampling tube connector 12 and the respiratory therapy equipment, helps to shorten assembly time between the gas sampling tube 10 and respiratory therapy equipment, further shortens manufacturing time of the respiratory therapy equipment, and improves production capacity of the respiratory therapy equipment.

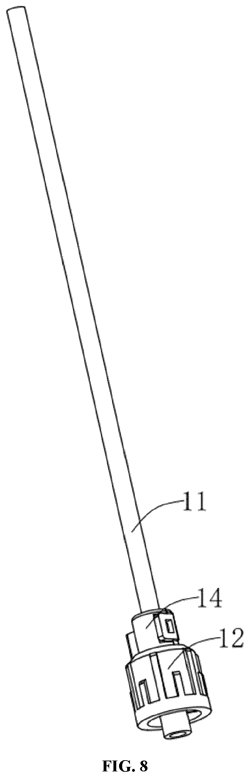

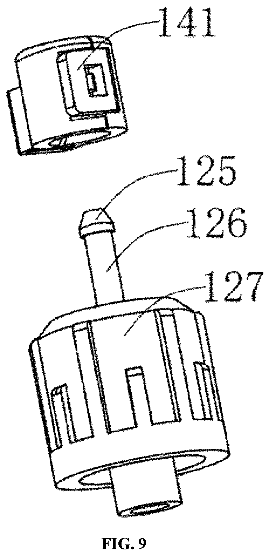

Referring to FIG. 8 to FIG. 10, in some embodiments, the gas sampling tube connector has a second inverted fastener 125, a second tube entrance piece 126, and a second sleeving piece 127. The second inverted fastener 125, the second tube entrance piece 126, and the second sleeving piece 127 are arranged in sequence from top to bottom. The second sleeving piece 127 is sleeved over the respiratory therapy equipment. The second tube entrance piece 126 is detachably connected to the gas delivery tube. The locking fastener 14 is arranged as a clamping locking fastener 141. The clamping locking fastener 141 is provided with a first clamping piece 1411, a second clamping piece 1412, and a deformation connecting part 1413 in sequence. The first clamping piece 1411 and the second clamping piece 1412 may be clamped with each other. Each of the first clamping piece 1411 and the second clamping piece 1412 is provided with a semicircular groove 1414, and the semicircular groove 1414 is adapted to the gas delivery tube in shape. The deformation connecting part 1413 is rotatably connected to the first clamping piece 1411 and the second clamping piece 1412. The clamping locking fastener 141 is configured to fix the gas delivery tube to interiors of the second inverted fastener and the second tube entrance piece, so that a traditional glue curing process is not relied any longer, and the problem of release of volatile organic matters caused by glue evaporation is solved. A stable mechanical connection of the gas delivery tube can be achieved through cooperation between the first inverted fastener and the clamping locking fastener 141 and between the first tube entrance piece and the clamping locking fastener 141, so that reliability and air tightness of assembly between the gas delivery tube and the gas sampling tube connector are improved, and meanwhile, a problem of a loosen connection caused by incomplete glue curing and glue aging is avoided.

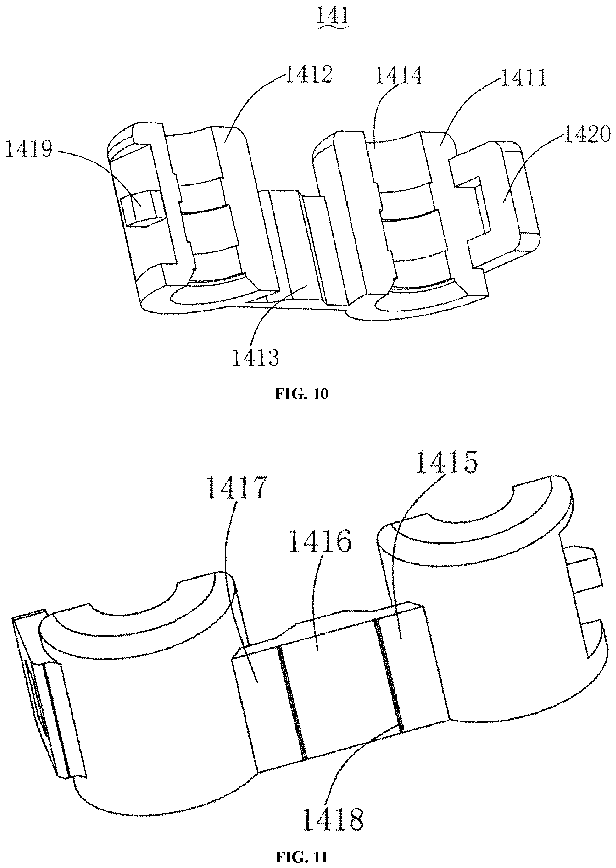

Referring to FIG. 11, in some embodiments, the deformation connecting part 1413 is provided with a first connecting part 1415, a deformation main body 1416, and a second connecting part 1417. The first connecting part 1415 is fixedly connected to the first clamping piece 1411, the second connecting part 1417 is fixedly connected to the second clamping piece 1412, and each of the connecting part 1415 and the second connecting part 1417 is rotatably connected to the deformation main body 1416. In a case that the gas delivery tube is assembled or disassembled, the first clamping piece 1411 and the second clamping piece 1412 can be rotated more conveniently, assembly difficulty is reduced, assembly efficiency is improved, and meanwhile, damage to the gas delivery tube when excessive stress is applied during assembling is avoided, and service life of the gas sampling tube is prolonged.

Referring to FIG. 11, in some embodiments, a bending groove 1418 is provided at a rotatable connection position between the first connecting part 1415 and the deformation main body 1416 and between the second connecting part 1417 and the deformation main body 1416, which helps to enhance flexibility and accuracy of the first clamping piece 1411 and the second clamping piece 1412 during rotating, so that deformation actions can be completed more smoothly when the first clamping piece 1411 and the second clamping piece 1412 are assembled or disassembled, and a risk of damage to the clamping locking fastener 141 caused by non-uniform deformation or stress concentration of the first clamping piece 1411 and the second clamping piece 1412 is reduced. Overall assembly quality and stability of the gas sampling tube are further improved greatly.

Referring to FIG. 10, in some embodiments, a clamping bulge 1419 is provided on the first clamping piece 1411, and a clamping buckle 1420 is arranged on the second clamping piece 1412. The clamping buckle 1420 is buckled in the clamping bulge 1419 by rotating the second clamping piece 1412 in a case that the semicircular groove 1414 of the first clamping piece 1411 fits the gas delivery tube, so that the gas delivery tube can be ensured to be firmly fixed to the inverted fastener and the tube entrance piece during rotating the second clamping piece 1412. In a buckling manner of buckling the clamping buckle 1420 into the clamping bulge 1419, the gas delivery tube can be effectively prevented loosening or falling off during use, continuity and accuracy of gas sampling are ensured, a firm and reliable mechanical connection is achieved without using glue, a traditional glue curing process is further not used and relied, and a problem of release of volatile organic matters caused by glue volatilization is avoided.

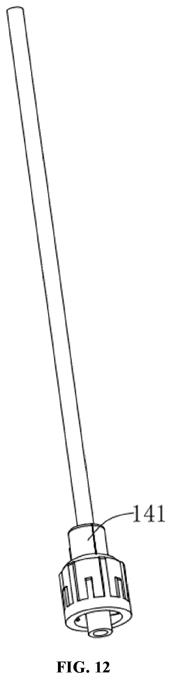

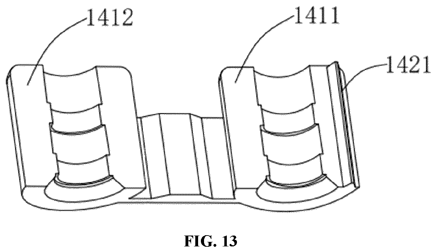

Referring to FIG. 12 and FIG. 13, in some embodiments, an outer side surface of the first clamping piece 1411 corresponding to the second clamping piece 1412 is provided with a second ultrasonic line 1421. The second ultrasonic line 1421 is configured to perform ultrasonic welding on the first clamping piece 1411 and the second clamping piece 1412, so that strength of a connection between the first clamping piece 1411 and the second clamping piece 1412 is further enhanced, air tightness and firmness of the connection between the first clamping piece 1411 and the second clamping piece 1412 are ensured, and durability and safety of the gas sampling tube under high-frequency use conditions are ensured.

A second embodiment of the present disclosure provides respiratory therapy equipment, including the glue-free chemical solvent-free gas sampling tube as described above, so that air tightness and welding quality between the gas sampling tube connector and the connector locking fastener are greatly improved, a problem of loosening that may be caused by a traditional glue connection is avoided, a curing process using glue is abandoned, a safety risk caused by volatilization of volatile organic matters is eliminated, user safety is ensured, moreover, air-drying curing time is not required, manufacturing time and cost for the respiratory therapy equipment are reduced, and production capacity of products of the respiratory therapy equipment is improved.

In conclusion, the present disclosure provides a glue-free chemical solvent-free gas sampling tube and respiratory therapy equipment. The gas sampling tube includes: a gas delivery tube; a gas sampling tube connector, where the gas delivery tube is arranged on the gas sampling tube connector; and a locking fastener, where the locking fastener is configured to fix the gas delivery tube to the gas sampling tube connector. The gas sampling tube replaces in a solution of connecting the gas sampling tube connector and the gas delivery tube through glue or a chemical solvent in a conventional art by achieving a stable connection between the gas sampling tube connector and the gas delivery tube through the additionally arranged locking fastener, and then a novel glue-free chemical solvent-free gas sampling tube is provided, so that release of volatile organic matters is avoided fundamentally, a production process is simplified, safety and reliability of a product are significantly improved, meanwhile, the gas sampling tube connector is mechanically connected to the gas delivery tube by using the locking fastener without waiting for glue to air-dry and cure, manufacturing time of the gas sampling tube is shortened, and production capacity of the gas sampling tube is improved.

It is to be understood that applications of the present disclosure are not limited to the foregoing examples. For those of ordinary skill in the art, improvements or transformations can be made according to the foregoing descriptions, and all these improvements and transformations fall within the scope of protection of the claims attached to the present disclosure.

## Claims

1. A glue-free chemical solvent-free gas sampling tube, configured for respiratory therapy equipment, comprising:
a gas delivery tube;
a gas sampling tube connector, wherein the gas delivery tube is arranged on the gas sampling tube connector; and
a locking fastener, wherein the locking fastener is configured to fix the gas delivery tube to the gas sampling tube connector.

2. The glue-free chemical solvent-free gas sampling tube according to claim 1, wherein the gas sampling tube connector has a first inverted fastener, a first tube entrance piece, a locking part, and a first sleeving piece arranged in sequence from top to bottom; the first sleeving piece is sleeved over the respiratory therapy equipment, and the first tube entrance piece is detachably connected to the gas delivery tube; and
the locking fastener is arranged as a connector locking fastener, the connector locking fastener is provided with a perforation hole, a plurality of fixing pieces are fixedly arranged on the connector locking fastener, and the plurality of fixing pieces are configured to cooperate with the locking part and the gas sampling tube connector to fix the gas delivery tube to an interior of the first tube entrance piece.

3. The glue-free chemical solvent-free gas sampling tube according to claim 2, wherein each of the plurality of locking parts comprises:
a propping boss, wherein the propping boss is provided below the first tube entrance piece, and the propping boss is provided above the first sleeving piece; and
an anti-stuck bulge, wherein the anti-stuck bulge is provided at an end of the propping boss deviating from the first tube entrance piece, and the anti-stuck bulge is integrally formed with the first sleeving piece and the propping boss; and the anti-stuck bulge is configured to partition a space between the propping bulge and the first sleeving piece into a clamping area.

4. The glue-free chemical solvent-free gas sampling tube according to claim 3, wherein an end of the propping boss close to the first tube entrance piece is provided with an expansion part, and the expansion part is configured to expand the plurality of fixing pieces outwards; and an included angle between a tangent line of the expansion part and an axis of the gas sampling tube connector is set as 20°-80°.

5. The glue-free chemical solvent-free gas sampling tube according to claim 4, wherein each of the plurality of fixing pieces comprises a plurality of clinching fasteners, and the plurality of clinching fasteners are fixedly arranged at an end of the connector locking fastener deviating from the perforation hole; and each of the plurality of fixing pieces comprises a deformation part and a buckle part.

6. The glue-free chemical solvent-free gas sampling tube according to claim 5, wherein the buckle part is integrally provided with a propping section, a side support section, and a locking section, and an included angle between a tangent line of the propping section and a tangent line of the locking section is set as 10°-90°, wherein the gas delivery tube penetrates out from the perforation hole, the gas delivery tube is sleeved in the first inverted fastener and the first tube entrance piece, the propping section is propped against the expansion part, the expansion part expands outwards to drive the deformation part to bend, and the side support section is propped against a side wall of the propping boss to lock the locking section in the clamping area.

7. The glue-free chemical solvent-free gas sampling tube according to claim 2, wherein the connector locking fastener further comprises:
a first ultrasonic line, wherein the first ultrasonic line is annularly arranged at an end of the connector locking fastener deviating from the perforation hole, and the first ultrasonic line is configured to perform ultrasonic welding with the gas sampling tube connector.

8. The glue-free chemical solvent-free gas sampling tube according to claim 7, wherein a cross section of the first ultrasonic line along an axial direction of the connector locking fastener is arranged in a conical shape, a triangle, or a cone shape.

9. The glue-free chemical solvent-free gas sampling tube according to claim 8, wherein a diameter of the first ultrasonic line is set as 1.1 times-2 times that of the perforation hole.

10. The glue-free chemical solvent-free gas sampling tube according to claim 7, wherein each of the plurality of locking parts comprises a locking boss, and the locking boss is fixedly provided above the first tube entrance piece; and
each of the plurality of fixing pieces comprises a plurality of rotary fasteners, the plurality of rotary fasteners are fixedly arranged on an inner side wall of the connector locking fastener, and the plurality of rotary fasteners are configured to cooperate with the locking boss and the gas sampling tube connector to fix the gas delivery tube to the first tube entrance piece, wherein
the gas delivery tube penetrates out from the perforation hole, the gas delivery tube is sleeved in the first inverted fastener and the first tube entrance piece, the connector locking fastener is propped against the locking boss, the plurality of rotary fasteners are rotated into the locking boss by rotating the connector locking fastener, and ultrasonic welding is performed on the locking boss and the gas sampling tube connector by using the first ultrasonic line.

11. The glue-free chemical solvent-free gas sampling tube according to claim 1, wherein the gas sampling tube connector has a second inverted fastener, a second tube entrance piece, and a second sleeving piece arranged in sequence from top to bottom; the second sleeving piece is sleeved over the respiratory therapy equipment, and the second tube entrance piece is detachably connected to the gas delivery tube;
the locking fastener is arranged as a clamping locking fastener, wherein the clamping locking fastener is provided with a first clamping piece, a second clamping piece, and a deformation connecting part in sequence, each of the first clamping piece and the second clamping piece is provided with a semicircular groove, and a shape of the semicircular groove is adapted to the gas delivery tube; the deformation connecting part is rotatably connected to the first clamping piece and the second clamping piece; and the clamping locking fastener is configured to fix the gas delivery tube to interiors of the second inverted fastener and the second tube entrance piece.

12. The glue-free chemical solvent-free gas sampling tube according to claim 11, wherein the deformation connecting part is provided with a first connecting part, a deformation main body, and a second connecting part, the first connecting part is fixedly connected to the first clamping piece, the second connecting part is fixedly connected to the second clamping piece, and each of the connecting part and the second connecting part is rotatably connected to the deformation main body.

13. The glue-free chemical solvent-free gas sampling tube according to claim 12, wherein a bending groove is provided at a rotatable connection position between the first connecting part and the deformation main body and between the second connecting part and the deformation main body.

14. The glue-free chemical solvent-free gas sampling tube according to claim 11, wherein a clamping bulge is provided on the first clamping piece, and a clamping buckle is arranged on the second clamping piece, wherein the clamping buckle is buckled in the clamping bulge by rotating the second clamping piece in a case that the semicircular groove of the first clamping piece fits the gas delivery tube;
alternatively, an outer side surface of the first clamping piece corresponding to the second clamping piece is provided with a second ultrasonic line, and the second ultrasonic line is configured to perform ultrasonic welding on the first clamping piece and the second clamping piece.

15. Respiratory therapy equipment, comprising the glue-free chemical solvent-free gas sampling tube according to any one of claims 1 to 14.
